# EUROPÄISCHE PATENTANMELDUNG

(11) **EP 2 070 558 A2**
(43) Veröffentlichungstag der Anmeldung: **17.06.2009**
(21) Anmeldenummer: 08168794.9
(22) Anmeldetag: 11.11.2008
(51) Int. Cl.: A61L 27/54, A61L 27/28, A61L 29/08, A61L 29/16, A61L 31/08, A61L 31/16

(54) **Implantate mit membrandiffusionskontrollierter Wirkstofffreisetzung**

(30) Priorität: 10.12.2007 DE 102007059755
(71) Anmelder: BIOTRONIK VI Patent AG, 6341 Baar (CH); Universität Rostock, 18051 Rostock (DE); Universität Greifswald, 17487 Greifswald (DE)
(72) Erfinder: Schmitz, Klaus-Peter, 18119, Warnemünde (DE); Behrend, Detlef, 18119, Rostock (DE); Sternberg, Katrin, 18057, Rostock (DE); Grabow, Niels, 18055, Rostock (DE); Harder, Claus, 91080, Uttenreuth (DE); Klocke, Björn, 8006, Zürich (CH); Kroemer, Heyo, 17498, Neuenkirchen (DE); Weitschies, Werner, 17498, Neuenkirchen (DE)
(74) Vertreter: Lindner-Vogt, Karin L.

(57) **Zusammenfassung**

Die vorliegende Erfindung betrifft ein Implantat zur Implantierung in einen menschlichen oder tierischen Organismus umfassend oder bestehend aus einem Aufbau:
a) einem Implantatgrundkörper,
b) einer Primerschicht, die ganz oder teilweise auf der Oberfläche des Implantates aufgebracht ist,
c) einer Wirkstoffschicht bestehend aus ein, zwei, drei oder mehreren Wirkstoffen, die ganz oder teilweise auf der Oberfläche der Primerschicht b) aufgebracht ist und
d) einer diffusionskontrollierenden Schicht, die ganz oder teilweise auf die Wirkstoffschicht c) und gegebenenfalls die Primerschicht b) aufgebracht ist und die Diffusion des oder der Wirkstoffe der Wirkstoffschicht c) kontrolliert,
sowie ein Herstellungsverfahren für ein erfindungsgemäßes Implantat.

## Beschreibung

Die vorliegende Erfindung betrifft ein Implantat zur Implantation in einen menschlichen oder tierischen Organismus sowie ein Verfahren zur Herstellung eines erfindungsgemäßen Implantats.

Implantate sind Stoffe oder Teile, die zur Erfüllung bestimmter Ersatzfunktionen für einen begrenzten Zeitraum oder auf Lebenszeit in den menschlichen oder tierischen Körper eingebracht werden. Im Gegensatz zu Transplantaten bestehen Implantate aus künstlichem Material (Alloplastik). Es wird häufig nach medizinischen, plastischen oder funktionellen Implantaten unterschieden.

Medizinische Implantate haben die Aufgabe, Körperfunktionen zu unterstützen oder zu ersetzen. Je nach Funktion werden sie auch als implantierbare Prothese bezeichnet. Bekannte Vertreter sind z. B. Herzschrittmacher, Hirnschrittmacher bei Parkinson, Herzimplantate, Cochleaimplantate, Implantate in der Zahnmedizin, Stents und Implantate, die dazu dienen, ein Depot eines Arzneistoffes zu bilden sowie verschiedene Formen des Gelenkersatzes.

Plastische Implantate werden in der plastischen Chirurgie, z. B. zum Ersatz von zerstörten Körperteilen oder auch zur Veränderung von bestehenden Körperteilen verwendet.

Funktionelle Implantate dienen zur Überwachung von menschlichen oder tierischen Funktionen, indem z. B. RFID (radio frequency identicifaction) -Chips unter die Haut verpflanzt werden.

Aus der Vielzahl der vorhandenen Implantattypen kann man erkennen, dass Implantate und deren Anwendung in der Medizin einen großen Stellenwert erlangt haben.

Da bei klassischen Behandlungsprinzipien, wie beispielsweise bei der systemischen Applikation von ein oder mehreren Wirkstoffen, zum Teil erhebliche Nebenwirkungen, wie beispielsweise bei der Tumorbehandlung, zu erwarten sind, gewinnt eine lokale, kontrollierte Wirkstofffreisetzung am Zielort oder in der Nähe davon zunehmende Bedeutung (Local-Drug-Delivery-Konzept; LDD-Konzept). Um diese lokale Applikation von Wirkstoffen durchführen zu können, werden insbesondere Implantatgrundkörper mit Wirkstoffen beschichtet, die entweder am Zielort oder in dessen Nähe in einen menschlichen oder tierischen Organismus implantiert werden und so lokal Wirkstoffe freisetzen. Diese klinisch etablierte Methode wird jährlich weltweit millionenfach angewendet und es ist zu erwarten, dass bei Berücksichtigung der demoskopischen Verschiebung innerhalb der Alterspyramide der Bedarf an neuen Werkstoffen sowie neuen Darreichungsformen wächst.

Im orthopädischen Bereich sind im Zusammenhang von endoprothetischen Implantaten Implantat-assoziierte Infektionen und thromboembolische Komplikationen bekannt. Deshalb wird in diesem Bereich an wirkstoffbeschichteten, insbesondere antibiotikabeschichteten Implantaten geforscht.

Im Bereich der Herz-Kreislauf-Erkrankungen bieten minimalinvasive Therapieformen zur Aufweitung und Stabilisierung stenosierter Koronargefäße durch die Perkutane Transluminale Coronarangioplastie (PTCA) und die Stentimplantation eine sich immer weiter e-tablierende Behandlungsmethode. Um eine weitere Senkung der Spätkomplikationsrate, insbesondere der Wiederverengung des Gefäßes nach PCTA (sogenannte In-Stent-Restenose (ISR)), gerade bei Hochrisikopatienten zu erreichen, verfolgt die gegenwärtige Forschung das Ziel einer lokalen Wirkstoffadministration mittels eines medikamentenbeschichteten Stents (Drug Eluting Stents (DES)).

Von nicht zu unterschätzender Bedeutung sind die bei den bisher üblichen konventionellen Verfahren zum LDD-Konzept auftretende Komplikationsraten infolge von zum Teil Bolus-artiger Freisetzung des oder der Wirkstoffe am Applikationsort, was somit zu einer lokalen Überdosierung des oder der Wirkstoffe führen kann.

Bekannte Drug Eluting Stents umfassen üblicherweise einen Wirkstoff, der in einer Polymerbeschichtung dispergiert vorliegt bzw. innerhalb eines Layers einer Polymerbeschichtung dispergiert vorliegt, wobei die wirkstoffhaltige Polymerbeschichtung auf den Stentgrundkörper aufgebracht ist.

Eine solche Wirkstoff-inkorporierte Polymerschicht weist üblicherweise eine Schichtdicke von 10 bis 20 µm auf, sodass bei allseitiger Beschichtung der Stentstreben die Wandstärke des Stents um ungefähr das Doppelte erhöht wird. Verschiedene Untersuchungen, insbesondere die ISAR-STEREO-Studie, haben nun gezeigt, dass mit steigender Wandstärke das Restenoserisiko nach PCTA ebenfalls ansteigt.

Zudem kann es mit einer solchen Polymerbeschichtung schwierig sein, eine adäquate Pharmakokinetik des oder der Wirkstoffe am Zielort im menschlichen oder tierischen Organismus zu erreichen.

In WO 2006/118937 A2 werden Stents offenbart, die dahingehend ausgestaltet sind, dass die Wirkstofffreisetzung von in Polymermatrices eingebetteten Wirkstoffen kontrolliert wird.

In US 6,753,071 B1 werden implantierbare Medizinprodukte, insbesondere Stents, offenbart, die eine Beschichtung aufweisen, welche die Freisetzungsrate des in eine Polymerschicht inkorporierten Wirkstoffs reduziert.

Die Aufgabe der vorliegenden Erfindung besteht nun darin, ein Implantat zur Implantation in einen menschlichen oder tierischen Organismus bereitzustellen, das verbesserte physiologische und/oder physiko-chemische Eigenschaften, insbesondere eine verbesserte kontrollierte Wirkstofffreisetzung ermöglicht.

Die vorliegende Aufgabe wird durch die Gegenstände der unabhängigen Ansprüche gelöst. Bevorzugte Ausgestaltungen werden in den abhängigen Ansprüchen sowie der nachfolgenden Beschreibung dargestellt.
Die vorliegende Aufgabe wird dementsprechend gelöst durch ein Implantat zur Implantation in einen menschlichen oder tierischen Organismus umfassend oder bestehend aus einem Aufbau:
a) einem Implantatgrundkörper,
b) einer Primerschicht, die ganz oder teilweise auf der Oberfläche des Implantates aufgebracht ist,
c) einer Wirkstoffschicht bestehend aus ein, zwei, drei oder mehreren Wirkstoffen, die ganz oder teilweise auf der Oberfläche der Primerschicht b) aufgebracht ist und
d) einer diffusionskontrollierenden Schicht, die ganz oder teilweise auf die Wirkstoffschicht c) und gegebenenfalls die Primerschicht b) aufgebracht ist und die Diffusion des oder der Wirkstoffe der Wirkstoffschicht c) kontrolliert.

Ein weiterer Gegenstand der vorliegenden Erfindung betrifft ein Verfahren zur Herstellung eines erfindungsgemäßen Implantates, dadurch gekennzeichnet, dass
a) folgende Materialien bereit gestellt werden
   i. ein Implantatgrundkörper,
   ii. eine Zubereitung für die Primerschicht umfassend ein oder mehrere Primermaterialien,
   iii. eine Zubereitung für die Wirkstoffschicht umfassend oder bestehend aus ein, zwei, drei oder mehreren Wirkstoffen und
   iv. eine Zubereitung für die diffusionskontrollierende Schicht umfassend oder bestehend aus ein oder mehreren Polymeren sowie einem oder mehreren Lösungsmitteln,
b) der Implantatgrundkörper i) ganz oder teilweise mit der Zubereitung für die Primerschicht ii) beschichtet wird,
c) anschließend der ganz oder teilweise mit Primer beschichtete Implantatgrundkörper aus b) ganz oder teilweise mit der Zubereitung für die Wirkstoffschicht iii) beschichtet wird, und
d) anschließend der ganz oder teilweise mit wirkstoffhaltiger Schicht beschichtete Implantatgrundkörper aus c) ganz oder teilweise mit der Zubereitung für die diffusionskontrollierende Schicht iv) beschichtet wird.

Die vorliegende Erfindung beruht auf der Erkenntnis, dass durch den erfindungsgemäßen Schichtaufbau des erfindungsgemäßen Implantats eine geringere Schichtdicke für die Wirkstoffschicht und damit eine geringere Gesamtschichtdicke, vorzugsweise Wandstärke für einen erfindungsgemäßen Stent, bei gleichbleibenden oder verbesserten Haftungseigenschaften erreicht wird. Zudem ermöglicht die diffusionskontrollierenden Schicht d) eine kontrollierte Wirkstofffreisetzung, so dass lokale Überdosierungen, insbesondere bolusartige Wirkstofffreisetzungen des oder der Wirkstoffe vermieden werden.

Aufgrund der speziell verwendeten Primermaterialien wird die erfindungsgemäße Beschichtung auf nahezu allen Implantatoberflächen ermöglicht. Somit wird die medizinische Versorgung Aufgrund einer verbesserten LDD-Therapie ebenfalls verbessert.
Demnach ermöglicht ein erfindungsgemäßes Implantat eine optimierte, da kontrollierte, Freisetzung von ein oder mehreren Wirkstoffen durch die diffusionskontrollierende Schicht bei gleichzeitiger Optimierung der Therapie oder Prophylaxe, insbesondere bei Stents durch Minimierung des Restenoserisikos durch Verringerung der Wandstärke bei dem erfindungsgemäßen Stent.

Implantate bzw. Implantatgrundkörper im Sinne dieser Erfindung können alle medizinischen Implantate bzw. Implantatgrundkörper darstellen und werden beispielsweise ausgewählt aus der Gruppe bestehend aus Herzschrittmacher; Hirnschrittmacher; Herzimplantate; Schrittmacherelektroden, Defibrillationselektroden, Cochleaimplantate; Implantate für die Zahnmedizin; Depotimplantate, die dazu dienen, ein Depot eines Wirkstoffs zu bilden; biodegradierbare oder dauerhafte, koronare oder periphere Stents; biodegradierbare oder dauerhafte Stents für andere Hohlräume, vorzugsweise die Speiseröhre, den Gallengang, die Harnröhre, die Prostata oder die Luftröhre; und local drug delivery (LDD)-Implantate, die vorzugsweise endovaskulär im Blut oder anderen Hohlräumen implantiert werden sein. Bevorzugte Stents sind dauerhafte oder biodegradierbare koronare Stents (z.B. Koronarstent) und hiervon sind biodegradierbare Metallstents besonders bevorzugt.

Neben diesen Anforderungen sollen die ursprünglichen mechanischen Funktionen eines Koronarstents, die Dilatierbarkeit, der geringe Recoil, die Stabilität über eine gewünschte Zeitdauer (im Falle degradierbarer Stents, z.B. bestehend aus Magnesium und seinen Legierungen) sowie die Flexibilität beibehalten werden.

Im Folgenden werden erfindungsgemäß zu verwendende Implantat-Materialien sowie bevorzugte Ausgestaltungen hiervon beschrieben:

### Biodegradierbarer Implantatgrundkörper, insbesondere biodegradierbarer Stent:

Im Sinne der vorliegenden Erfindung bedeutet "biodegradierbarer Implantatgrundkörper", insbesondere "biodegradierbarer Stent", dass der Grundkörper in physiologischer Umgebung, insbesondere im Gefäßsystem eines menschlichen oder tierischen Körpers degradiert, also so abgebaut wird, dass der Stent seine Integrität verliert. Vorzugsweise degradieren biodegradierbare Implantatgrundkörper erst dann, wenn die Funktion des Implantates nicht mehr physiologisch sinnvoll bzw. notwendig ist. Bei biodegradierbaren Stents bedeutet das, dass der Stent vorzugsweise erst dann degradiert oder seine Integrität verliert, wenn das traumatisierte Gewebe des Gefäßes verheilt ist und der Stent somit nicht länger im Gefäßlumen verbleiben muss.

### Metallische Grundkörper:

Vorzugsweise ist der biodegradierbare Werkstoff ein metallischer Werkstoff, der eine biokorrodierbare Legierung darstellt, wobei die Hauptkomponente der Legierung ausgewählt wird aus der Gruppe Magnesium, Eisen, Zink und Wolfram; insbesondere wird für einen degradierbaren metallischen Werkstoff eine Magnesiumlegierung bevorzugt.

Die Legierung, insbesondere umfassend Magnesium, Eisen, Zink und/oder Wolfram, ist so in ihrer Zusammensetzung zu wählen, dass sie biokorrodierbar ist. Als biokorrodierbar im Sinne der vorliegenden Erfindung werden Legierungen bezeichnet, bei denen in physiologischer Umgebung ein Abbau stattfindet, der letztendlich dazu führt, dass der gesamte Stent oder der aus dem Werkstoff gebildete Teil des Stents seine mechanische Integrität verliert. Unter Legierung wird vorliegend ein metallisches Gefüge verstanden, deren Hauptkomponente Magnesium, Eisen, Zink oder Wolfram ist. Hauptkomponente ist die Legierungskomponente, deren Gewichtsanteil an der Legierung am Höchsten ist. Ein Anteil der Hauptkomponente beträgt vorzugsweise mehr als 50 Gew.%, weiter bevorzugt mehr als 70 Gew.%. Eine Magnesiumlegierung ist bevorzugt.

Ist der Werkstoff eine Magnesiumlegierung, so enthält diese vorzugsweise Yttrium und weitere Seltenerdmetalle, da sich eine derartige Legierung aufgrund ihrer physikochemischen Eigenschaften und ihrer hohen Biokompatibilität, insbesondere auch ihrer Abbauprodukte, auszeichnet.
Besonders bevorzugt werden Magnesiumlegierungen der WE-Reihe, insbesondere WE43 sowie Magnesiumlegierungen der Zusammensetzung Seltenerdmetalle 5,5 - 9,9 Gew.%, davon Yttrium 0,0 - 5,5 Gew.% und Rest < 1 Gew.%, der Zirkonium und/oder Silizium enthalten kann, wobei Magnesium den auf 100 Gew.% fehlenden Anteil an der Legierung einnimmt, eingesetzt. Diese Magnesiumlegierungen bestätigten bereits experimentell und in ersten klinischen Versuchen ihre besondere Eignung, d.h. sie zeigen eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 an Lanthan (57) folgenden Elemente, nämlich Cer (58), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71), verstanden.

### Polymer-Grundkörper:

Implantatgrundkörper, insbesondere Stentgrundkörper, können ebenfalls aus biodegradierbarem Polymer bestehen, vorzugsweise aus Polydioxanon; Polyglycolid; Polycaprolacton; Polyhydroxyvaleriansäure, Polyhydroxybuttersäure, Polylactide, vorzugsweise Poly(L-lactid), Poly(D-lactid), Poly(D,L-lactid) und Blends sowie Copolymere, und vorzugsweise Poly(L-lactid-co-glycolid), Poly(D,L-lactid-co-glycolid), Poly(L-lactid-co-D,L-lactid), Poly(L-lactid-co-trimethylencarbonat) und Tri-Blockcopolymere; Polysaccharide, vorzugsweise Chitosan, Levan, Hyaluronsäure, Heparin, Dextran und Cellulose.

### Dauerhafter Implantatgrundkörper, vorzugsweise dauerhafter Stentgrundkörper:

Im Gegensatz zum biodegradierbaren Grundkörper wird der "dauerhafte Implantatgrundkörper", vorzugsweise der "dauerhafte Stentgrundkörper" in physiologischer Umgebung im menschlichen oder tierischen Körper im Wesentlichen nicht abgebaut, so dass er seine Integrität behält.

In einem weiteren Ausführungsbeispiel besteht der Grundkörper eines dauerhaften Implantats, insbesondere eines dauerhaften Stents, aus einem Formgedächtnis-Material aus einem oder mehreren Materialien ausgewählt aus der Gruppe der Nickel-Titan-Legierungen und Kupfer-Zink-Aluminium-Legierungen, vorzugsweise aus Nitinol.

In einem weiteren bevorzugten Ausführungsbeispiel besteht der Grundkörper eines dauerhaften Implantates, insbesondere eines Stents, aus Edelstahl, vorzugsweise aus einem Cr-Ni-Fe-Stahl - hier bevorzugt die Legierung 316L - oder einem Co-Cr-Stahl.

Ferner kann der Grundkörper des Stents mindestens teilweise aus Kunststoff, vorzugsweise Polyetherurethan, und/oder einer Keramik bestehen.

Werden im Sinne der vorliegenden Erfindung endovaskulär implantierbare Stents als implantierbare Grundkörper verwendet, so können alle üblichen Stentgeometrien verwendet werden. Insbesondere bevorzugt sind Stentgeometrien, die insbesondere in US 6,896,695, US 2006/241742, US 5,968,083 (Tenax), EP 1 430 854 (Helix-Design), US 6,197,047 und EP 0 884 985 beschrieben werden.

Die bevorzugten Ausgestaltungen des erfindungsgemäß verwendbaren Implantatgrundkörpers können in allen denkbaren Varianten untereinander, aber auch mit den weiteren hierin offenbarten bevorzugten Ausgestaltungen kombiniert werden.

Im Sinne der vorliegenden Erfindung können für die Primerschicht ein oder mehrere übliche Materialien ausgewählt werden, wobei die Materialien üblicherweise keine bzw. keine wesentliche Wechselwirkung mit dem oder den Wirkstoffen der Wirkstoffschicht eingehen. Sofern es sich bei der diffusionskontrollierenden Schicht um eine degradierbare Schicht handelt, verhindert oder reduziert die Primerschicht, nach Implantation des erfindungsgemäßen Implantats und während oder nach Degradierung der diffusionskontrollierenden Schicht, den Austritt von Schwermetallionen aus dem Implantatgrundkörper. Zudem wird die Primerschicht bevorzugt so ausgewählt, dass sie mit der diffusionskontrollierenden Schicht eine Haftung ausbilden kann.

In einer bevorzugten Ausgestaltung der vorliegenden Erfindung wird deshalb die Primerschicht ausgewählt aus einem oder mehreren Materialien aus der Gruppe bestehend aus Siliziumkarbid (SiC), Diamond-Like Carbon (DLC), pyrolytischem Kohlenstoff (pyC), Parylene, Glycocalix und Hydroxylapatit oder Kombinationen hiervon. Parylene (Polypara-Xylene) werden erfindungsgemäße bevorzugt ausgewählt aus der Gruppe bestehend aus Parylene N, Parylene C und Parylene D (GALXYL®). Bevorzugt umfasst oder besteht die Primerschicht aus ein oder mehreren Materialien, die ausgewählt werden aus der Gruppe bestehend aus Siliziumkarbid (SiC), Diamond-Like Carbon (DLC), pyrolytischem Kohlenstoff (pyC) und Parylene.

Die Primerschicht wird üblicherweise ganz oder teilweise auf die Oberfläche des Implantatgrundkörpers aufgebracht. Sie vermittelt üblicherweise die Anhaftung des oder der Wirkstoffe der Wirkstoffschicht an den Implantatgrundkörper. Für den Fall, dass die Wirkstoffschicht erfindungsgemäß nicht auf der gesamten Oberfläche der Primerschicht aufgebracht ist, vermittelt die Primerschicht bevorzugt auch die Anhaftung der diffusionskontrollierenden Schicht an den Implantatgrundkörper.

Die bevorzugten Ausgestaltungen der erfindungsgemäß verwendbaren Primerschicht können in allen denkbaren Varianten untereinander, aber auch mit den weiteren hierin offenbarten bevorzugten Ausgestaltungen kombiniert werden.

Im Sinne der vorliegenden Erfindung werden die Polymere, insbesondere auch die molare Masse der Polymere, und die Schichtdicke der diffusionskontrollierenden Schicht üblicherweise so ausgewählt, dass sie eine bolusartige Freisetzung von Wirkstoffen verhindern und eine geeignete Wirkstofffreisetzung ermöglichen. Zusätzlich können Substanzen in die diffusionskontrollierende Schicht inkorporiert werden, die nach Implantation durch die Gewebsflüssigkeit aus der diffusionskontrollierenden Schicht herausgelöst werden, wodurch sich Diffusionskanäle bilden, durch welche die geeignete Wirkstofffreisetzung weiterhin ermöglicht wird.

In diesem Sinne werden üblicherweise für die erfindungsgemäßen Implantate Schichtdicken der diffusionskontrollierenden Schicht verwendet werden, die im Bereich von 5 µm bis 20 µm liegen, bevorzugt liegen sie im Bereich von 5 µm bis 10 µm. Diese Schichtdicken ergeben sich aus der Notwendigkeit, dass bevorzugt hydrophobe Polymere für die diffusionskontrollierende Schicht Verwendung finden sollen, die den notwendigen Wassereintrag in die Schicht in den gewünschten Zeiträumen im angegebenen Schichtdickenbereich für die Wirkstoffe zulassen.

Die molare Masse der Polymere der diffusionskontrollierenden Schicht liegt üblicherweise im Bereich von 100000 g/mol bis 1000000 g/mol, bevorzugt im Bereich von 300000 g/mol bis 700000 g/mol , weiter bevorzugt bei 400000 g/mol bis 650000 g/mol.

Wenn in die diffusionskontrollierende Schicht ein oder mehrere Substanzen inkorporiert werden, die dann nach Implantation aus der diffusionskontrollierenden Schicht herausgelöst werden, sind dies üblicherweise Substanzen, die unter physiologischen Bedingungen aus der diffusionskontrollierenden Schicht herausgelöst werden und so Diffusionskanäle durch die diffusionskontrollierenden Schicht bilden. Bevorzugt werden solche Substanzen ausgewählt aus der Gruppe bestehend aus Antiphlogistika (z. B. Dexamethason, Methylprednisolon), Cytostatika (z. B. Paclitaxel, Colchicin, Actinomycin D, Methotrexat) und Immunsuppressiva (z. B. Sirolimus, Pimecrolimus, Biolimus A9, Tacrolimus, Cyclosporin A, Mycophenolsäure), Thrombozytenaggregationshemmer (z. B. Abciximab, Iloprost), Wundheilungsförderer (z. B. 17β-Estradiol, Genistein) und Extrazelluläre Matrix-Modulatoren/Migrationshemmer (z. B. Tretinoin, MMP-Inhibitoren).

In einer bevorzugten Ausgestaltung der Erfindung wird die diffusionskontrollierende Schicht ausgewählt aus einem oder mehreren Polymeren umfassend oder bestehend aus Materialien, die ausgewählt werden aus der Gruppe bestehend aus Polyestern vorzugsweise Polydioxanon, Polyglycolid, Polycaprolacton, Polyhydroxyvaleriansäure, Polyhydroxybuttersäuren, Polylactide und Copolymere; Polyurethanen; Silikonen; Polyorganophosphazenen; Polymethacrylaten; Polystyren Block-Copolymeren, Polyethylen-covinylaceta; Polyethylenterephthalat sowie Blends oder Copolymere hiervon, sowie Phosphorylcholin-Polymeren und Polysacchariden, vorzugsweise Chitosan, Hyaluronsäure, Heparin, Dextran und Cellulose.

Bevorzugt werden ein oder mehrere Polymere der diffusionskontrollierenden Schicht erfindungsgemäß ausgewählt aus der Gruppe bestehend aus biodegradierbaren Polyestern (Polyhydroxyalkansäuren); Polydioxanon; Polyglycolid; Polycaprolacton; Polyhydroxyvaleriansäure; Polyhydroxybuttersäuren; Polylactide, vorzugsweise Poly(L-lactid), Poly(D-lactid), Poly(D,L-lactid) und Blends sowie Copolymere hiervon, wie vorzugsweise Poly(L-lactid-co-glycolid), Poly(D,L-lactid-co-glycolid), Poly(L-lactid-co-D,L-lactid), Poly-(L-lactid-co-trimethylencarbonat).

Im Sinne der vorliegenden Erfindung umhüllt die diffusionskontrollierende Schicht das erfindungsgemäße Implantat vollständig (Formschluss) oder teilweise. Für den Fall, dass die Wirkstoffschicht erfindungsgemäß teilweise, bevorzugt in Form von Wirkstoffinseln auf die Primerschicht aufgebracht ist, steht die diffusionskontrollierende Schicht bevorzugt auch mit der Primerschicht in Kontakt. Durch diesen Kontakt zwischen Primerschicht und diffusionskontrollierender Schicht besteht somit eine zusätzliche Haftung, die das Risiko des Abstreifens der diffusionskontrollierenden Schicht und gegebenenfalls der Wirkstoffschicht vom Implantat Aufgrund der auftretenden Reibungskräfte bei Implantation vermindern kann.

Polymere der diffusionskontrollierenden Schicht, die insbesondere gut an die Primermaterialien haften, werden erfindungsgemäß bevorzugt ausgewählt aus der Gruppe bestehend aus biodegradierbaren Polyestern (Polyhydroxyalkansäuren); Polydioxanon; Polyglycolid; Polycaprolacton; Polyhydroxyvaleriansäure; Polyhydroxybuttersäure; Polylactide, vorzugsweise Poly(L-lactid), Poly(D-lactid), Poly(D,L-lactid) und Blends sowie Copolymere hiervon, wie vorzugsweise Poly(L-lactid-co-glycolid), Poly(D,L-lactid-co-glycolid), Poly(L-lactid-co-D,L-lactid) und Poly-(L-lactid-co-trimethylencarbonat), die durch die durch die Ausbildung von van-der Waals-Kräften mit den Primermaterialien besonders gut in Haftung treten.

Die diffusionskontrollierende Schicht weist üblicherweise eine Schichtdicke im Bereich von 5 µm bis 20 µm auf, bevorzugt liegt sie aber im Bereich von 5 µm bis 10 µm.

Die bevorzugten Ausgestaltungen der erfindungsgemäß verwendbaren diffusionskontrollierenden Schicht können in allen denkbaren Varianten untereinander, aber auch mit den weiteren hierin offenbarten bevorzugten Ausgestaltungen kombiniert werden.

Ein Wirkstoff im Sinne dieser Erfindung ist eine Substanz, die im menschlichen oder tierischen Körper eine biologische Reaktion hervorruft. In diesem Sinne kann Wirkstoff auch synonym zu Arzneistoff und/oder Pharmakon verwendet werden.

Unter Wirkstoffschicht im Sinne der vorliegenden Erfindung ist sowohl eine vollständige Beschichtung der Primerschicht auf dem erfindungsgemäßen Implantatgrundkörper als auch eine teilweise Beschichtung der Primerschicht, beispielsweise in Form von Wirkstoffinseln, die unabhängig voneinander aus ein oder mehreren, gleichen oder unterschiedlichen Wirkstoffen bestehen, zu verstehen.

Bevorzugte Wirkstoffe für ein erfindungsgemäßes Implantat, werden ausgewählt aus der Gruppe bestehend aus Antiphlogistika, vorzugsweise Dexamethason, Methylprednisolon und Diclophenac; Cytostatika, vorzugsweise Paclitaxel, Colchicin, Actinomycin D und Methotrexat; Immunsuppressiva, vorzugsweise Limus-Verbindungen, weiter bevorzugt Sirolimus (Rapamycin), Zotarolimus (Abt-578), Tacrolimus (FK-506), Everolimus, Biolimus, insbesondere Biolimus A9 und Pimecrolimus, Cyclosporin A und Mycophenolsäure; Thrombozytenaggregationshemmer, vorzugsweise Abciximab und Iloprost; Statine, vorzugsweise Simvastatin, Mevastatin, Atorvastatin, Lovastatin, Pitavastatin und Fluvastatin; und Estrogene, vorzugsweise 17β-Estradiol, Daizein und Genistein; Lipidregulatoren, vorzugsweise Fibrate; Immunsuppressiva; Vasodilatatoren, vorzugsweise Satane; Calciumkanalblocker; Calcineurininhibitoren, vorzugsweise Tacrolimus; Antiinflammatorika, vorzugsweise Imidazole; Antiallergika; Oligonucleotide, vorzugsweise Decoy-Oligodesoxynukleotid (dODN); Endothelbildner, vorzugsweise Fibrin; Steroide; Proteine/Peptide; Proliferationshemmer; Analgetika und Antirheumatika. Besonders bevorzugt werden erfindungsgemäß Paclitaxel und Limus-Verbindungen, weiter bevorzugt Sirolimus (Rapamycin), Zotarolimus (Abt-578), Tacrolimus (FK-506), Everolimus, Biolimus, insbesondere Biolimus A9 und Pimecrolimus, ganz besonders bevorzugt Rapamycin (Sirolimus) verwendet.

Tabelle 1 zeigt eine Auflistung an Wirkstoffen, die erfindungsgemäß bevorzugt verwendet werden:

**Tabelle1: Übersicht über bevorzugte Wirkstoffe**

| **Antiproliferative und/oder antiinflammatorische Stoffe** | **Antithrombogene Stoffe** | **Migrationshemmer/ ECM-Modulatoren** | **Wundheilungs-/ Endothelialisie-rungsförderer** |
|---|---|---|---|
| Sirolimus | Hirudin | Probucol | 17β-Estradiol |
| Tacrolimus | Prostaglandine | Tretinoin | Genistein |
| Everolimus | Abciximab | Halofuginon | Statine |
| Biolimus A9 | | Prolylhydroxylase- | Tyrosinkinase- |
| Cyclosporin A | | Inhibitoren | Inhibitoren |
| Mycophenolsäure | | MMP-Inhibitoren | VEGF |
| Corticosteroide | | Batimastat | EPC-Antikörper |
| Diclophenac | | | NO-Donatoren |
| Natriumsalz | | | |
| Paclitaxel | | | |
| Colchicin | | | |
| Methotrexat | | | |
| Actinomycin D | | | |
| Tranilast | | | |
| Mitomycin | | | |
| QP-2 | | | |
| Vincristin | | | |
| Angiopeptin | | | |
| ABT 578 | | | |
| Pimecrolimus | | | |

Neben rein medizinischen Aspekten bestimmen Faktoren wie Molekulargewicht und Proteinbindungsvermögen des oder der Wirkstoffe wesentlich das Elutionsverhalten der Wirkstoffe in erfindungsgemäßen Implantaten. Besonders bevorzugt sind die oben beschriebenen Wirkstoffe, wenn sie ein Molekulargewicht im Bereich von 100 - 2000 Dalton aufweisen. Solche Wirkstoffe werden bevorzugt durch die erfindungsgemäße diffusionskontrollierende Schicht kontrolliert freigesetzt.

Die bevorzugten Ausgestaltungen der erfindungsgemäß verwendbaren Wirkstoffe können in allen denkbaren Varianten untereinander, aber auch mit den weiteren hierin offenbarten bevorzugten Ausgestaltungen kombiniert werden.

Ein weiterer Gegenstand der vorliegenden Erfindung ist ein Verfahren zur Herstellung eines erfindungsgemäßen Implantates.

Die zum erfindungsgemäßen Implantat beschriebenen Materialien, insbesondere für den Implantatgrundkörper, die Primerschicht, die Wirkstoffschicht sowie die diffusionskontrollierende Schicht inklusive der jeweils bevorzugten Ausgestaltungen können auch bevorzugt für das erfindungsgemäße Herstellungsverfahren verwendet werden.

Die erfindungsgemäß zu verwendende Zubereitung für die Primerschicht umfasst üblicherweise neben den erfindungsgemäß zu verwendenden Primermaterialien auch ein oder mehrere Lösungsmittel. Die Beschichtung der Oberfläche des Implantatgrundkörpers mit der Zubereitung für die Primerschicht wird mittels üblicher Verfahren, beispielsweise Physikalische Gasphasenabscheidung (PVD) (bevorzugt für DLC), Chemische Gasphasenabscheidung (CVD) (bevorzugt für SiC und Parylene) aufgebracht. Für die Ausgestaltung, dass der Implantatgrundkörper ein - wie vorstehend beschriebener - erfindungsgemäßer Stentgrundkörper ist, wird die Primerschicht bevorzugt auf der abluminalen Seite des Stentgrundkörpers oder Teilen davon - d.h. der Oberfläche oder den Teilen der Oberfläche des Stentgrundkörpers, die mit dem menschlichen oder tierischen Gewebe der Gefäße oder Organe in Kontakt steht - aufgetragen. Dies kann bevorzugt dadurch bewerkstelligt werden, dass die luminale Oberfläche des Stentgrundkörpers - also die Oberfläche des Stentgrundkörpers oder zumindest Teile davon, die mit dem Lumen eines Gefäßes oder eines anderen Hohlraumes des menschlichen oder tierischen Körpers in Kontakt steht - beispielsweise durch Aufziehen eines Stents auf einen Zylinder, einen Dorn, eine Kanüle etc., so geschützt wird, dass die luminale Oberfläche nicht mit der Zubereitung der Primerschicht in Kontakt kommt und somit nicht damit beschichtet wird.

Die Wirkstoffbeschichtung der Oberfläche oder Teile der Oberfläche des Implantatgrundkörpers, bevorzugt Stentgrundkörpers, insbesondere mit Primerschicht wird beispielsweise mittels üblicher Verfahren durchgeführt: Tauchverfahren (dip coating), wobei der Stentgrundkörper auf einen Dorn aufgesteckt ist, Sprühbeschichtung mit Ein- bzw. Mehrstoffdüse, Rotationszerstäubung und Druckdüsen, Sputtern etc..

Der oder die Wirkstoffe der Wirkstoffschicht können in Form einer Wirkstoffschmelze oder als Wirkstoff-Lösungsmittelgemisch mittels der üblichen Verfahren aufgetragen werden. Übliche Verfahren für ein Wirkstoff-Lösungsmittelgemisch sind: Tauchverfahren (dip coating), wobei der Stentgrundkörper auf einen Dorn aufgesteckt ist, Sprühbeschichtung mit Ein- bzw. Mehrstoffdüse, Rotationszerstäubung und Druckdüsen, Sputtern etc..

Sofern ein Wirkstoff-Lösungsmittelgemisch vorliegt, sollte das Lösungsmittel vor der Beschichtung mit der diffusionskontrollierenden Schicht durch übliche Verfahren, insbesondere Trocknung oder andere physikalische Verfahren, entfernt werden. Dies dient insbesondere dazu, dass die Polymere der diffusionskontrollierenden Schicht mit dem oder den Wirkstoffen im Wesentlichen nicht gemischt werden und so die Wirkstoffmoleküle der Wirkstoffschicht (homogen) dispergiert in der diffusionskontrollierenden Schicht vorliegen würden.

Für eine genaue Dosierung des oder der Wirkstoffe der Wirkstoffschicht kann die Wirkstoffschmelze oder die Wirkstofflösung auf den Implantatgrundkörper, bevorzugt Stentgrundkörper, vorzugsweise mittels üblicher Pipetiertechniken aufgetragen werden. Sofern der Implantatgrundkörper, bevorzugt Stentgrundkörper, Einkerbungen in Form von Dellen, Körbchen etc. aufweist, wird die wirkstoffhaltige Zubereitung bevorzugt in die Einkerbungen, insbesondere die Dellen, Körbchen etc., mittels eines Wirkstoffstrahles dosiert.

Die Zubereitung für die diffusionskontrollierende Schicht umfasst oder besteht aus den oben beschriebenen Polymeren inklusive der bevorzugten Ausgestaltungen sowie einem oder mehreren Lösungsmitteln.

Die Beschichtung der Oberfläche oder Teile der Oberfläche des Implantatgrundkörpers, bevorzugt Stentgrundkörpers wird ebenfalls üblicherweise mittels Tauchverfahren (dip coating), wobei der Stentgrundkörper auf einen Dorn aufgesteckt ist, Sprühbeschichtung mit Ein- bzw. Mehrstoffdüse, Rotationszerstäubung und Druckdüsen, Sputtern etc., vorgenommen.

Üblicherweise werden die Lösungsmittel in der Zubereitung für die diffusionskontrollierende Schicht so gewählt, dass sie den oder die verwendeten Wirkstoffe der Wirkstoffschicht zumindest teilweise nicht lösen, so dass keine bzw. keine wesentliche Vermischung der diffusionskontrollierenden Schicht mit den gelösten Wirkstoffverbindungen stattfindet.

Die vorstehend beschriebenen Beschichtungsschritte mit der Primerzubereitung, der wirkstoffhaltigen Zubereitung und/oder der diffusionskontrollierenden Zubereitung können gegebenenfalls unabhängig voneinander nach dem jeweiligen Beschichtungsschritt durch geeignete Trocknungsschritte oder andere übliche physikalische oder chemische Nachbearbeitungsschritte weiterbehandelt werden.

### Figurenbeschreibung:

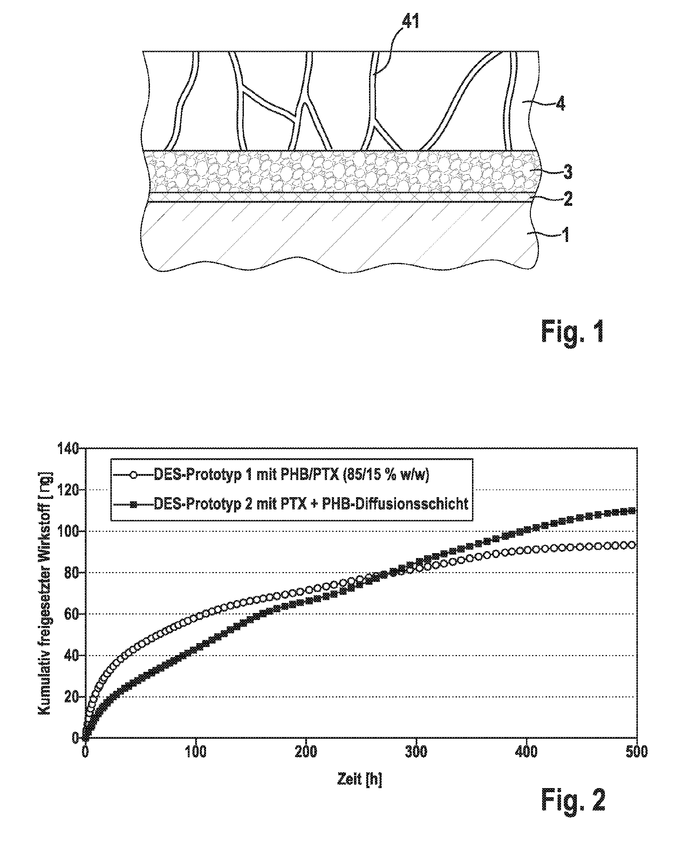

Die Figur 1 zeigt beispielhaft ein erfindungsgemäßes Implantat am Beispiel eines Querschnitts eines Stentsteges. Die vorliegende Erfindung wird allerdings nicht auf den hier gezeigten Aufbau beschränkt.

Figur 1: Ausschnitt eines Querschnitts eines Steges eines erfindungsgemäß beschichteten Stents.

Figur 2: Graphische Darstellung der in vitro Freisetzung von Paclitaxel (PTX) aus PHB-Stentbeschichtungen.

Figur 1 zeigt einen Ausschnitt eines Querschnitts eines Steges (1) eines erfindungsgemäß beschichteten Stents.

Weiterhin wird in Figur 1 die Primerschicht (2) dargestellt, die mit der Oberfläche des Implantatgrundkörpers sowie der Wirkstoffschicht (3) in Kontakt steht. Hierfür können die vorstehend beschriebenen Materialien verwendet werden und mittels üblicher Verfahren ganz oder teilweise auf die Oberfläche des Stents aufgetragen werden.

Zudem zeigt Figur 1, dass die Wirkstoffschicht (3) zwischen der Primerschicht (2) und der diffusionskontrollierenden Schicht (4) angeordnet ist. Wie vorstehend beschrieben, können ein, zwei, drei, vier oder mehrere Wirkstoffe verwendet werden, die mittels einer Wirkstoffschmelze oder eines Wirkstoff-Lösungsmittelgemisches mit üblichen Verfahren ganz oder teilweise auf die Oberfläche des Stentgrundkörpers insbesondere mit Primer aufgetragen werden können.

Weiterhin zeigt Figur 1, dass die diffusionskontrollierende Schicht (4) auf der Oberfläche der Wirkstoffschicht (3) angeordnet ist. Vorzugsweise ist die diffusionskontrollierende Schicht auf der gesamten Oberfläche der Wirkstoffschicht (3) angeordnet. Für den Fall, dass die Wirkstoffschicht (3) teilweise auf die Primerschicht (2), insbesondere in Form von Wirkstoffinseln (nicht dargestellt) aufgebracht ist, steht die diffusionskontrollierende Schicht (4) vorzugsweise auch in Kontakt mit der Primerschicht (2).

Die diffusionskontrollierende Schicht (4) kann Diffusionskanäle (41) umfassen, die bereits während der Beschichtung entstehen oder nachträglich unter physiologischen Bedingungen entstehen. Vorzugsweise sind die Diffusionskanäle so groß, dass die erfindungsgemäßen Wirkstoffe, insbesondere mit einem Molekulargewicht von 100 bis 2000 Dalton aus der Wirkstoffschicht (3) durch die diffusionskontrollierende Schicht (4) in den menschlichen oder tierischen Organismus diffundieren können.

Figur 2 zeigt eine in vitro Freisetzung von Paclitaxel (PTX) aus PHB-Stentbeschichtungen mit unterschiedlichem Schichtaufbau (jeweils n = 2) in PBS (37 °C); DES-Prototyp 1: PHB-Beschichtung mit Inkorporation von 15 % PTX (ca. 100 µg), DES-Prototyp 2: PTX-Grundschicht (ca. 100 µg) mit PTX-freier PHB-Deckschicht (etwa 150 µg).

Die in der Figurenbeschreibung dargestellten erfindungsgemäßen Ausgestaltungen, sind ebenfalls auf die vorstehende Beschreibung der Erfindung, insbesondere seiner bevorzugten Ausgestaltungen, anzuwenden.

### Ausführungsbeispiele

Die vorliegende Erfindung wird im Folgenden durch Ausführungsbeispiele beschrieben, die jedoch den Schutzumfang des erfindungsgemäßen Gegenstandes nicht limitieren.

### Ausführungsbeispiel1:

Es wurden CoCr-Stents mit einer Primerschicht (2-10 nm) auf der Basis von Siliziumkarbid verwendet. Diese zuvor mit einer Analysenwaage gewogenen Stents wurden mit einer 0.1 %igen acetonischen Paclitaxel (PTX)-Lösung im Sprühverfahren beschichtet. Für die Beschichtung der Stents im Sprühverfahren wurde eine elektro-pneumatische Beschichtungsanlage verwendet, mit der in einem automatisierten Arbeitsgang Stenttypen verschiedenster Dimensionen mit Polymer- und Wirkstoff-Lösungen beschichtet werden können Die Sprühparameter wurden so eingestellt, dass eine gleichmäßige PTX-Verteilung auf der Stentoberfläche resultierte. Danach wurde auf die PTX-Schicht durch Versprühen einer 0.16 %igen chloroformhaltigen Polyhydroxybuttersäure (PHB)-Lösung die diffusionskontrollierende, polymere, biodegradierbare Deckschicht aufgetragen. Daran schlossen sich die 15-minütige Trocknung der Stents bei Raumtemperatur und die mikroskopische Sichtkontrolle an. Nach der Lagerung der beschichteten Stents über 2 Tage im Vakuumtrockenschrank (6-8 mbar) bei 40 °C erfolgte wiederum die Wägung zur gravimetrischen Ermittlung der Schichtmasse. Zur Bestimmung der Schichtdicken wurden in kalthärtendes Epoxidharz eingebettete Stentmuster mit Hilfe lichtmikroskopischer und Bildanalyseverfahren ausgewertet. Die ermittelten Schichtdicken betrugen im Mittel 5 µm bei der Wirkstoffschicht und 10 µm bei der PHB-Schicht. Das in vitro Wirkstofffreisetzungsverhalten von Stents dieses Ausführungsbeispiels in Phosphatpuffer (PBS) bei 37 °C ist in der Figur 2 im Vergleich zur PHB-Beschichtung mit PTX-Inkorporation ohne eine äußere diffusionskontrollierende PHB-Beschichtung dargestellt.

Aus Figur 2 ist ersichtlich, dass bei Inkorporation des Wirkstoffs Paclitaxel in die Polymerschicht (DES-Prototyp 1) gegenüber der erfindungsgemäßen diffusionskontrollierenden Beschichtung (DES-Prototyp 2) eine bolusartige Freisetzung des Wirkstoffes zu verzeichnen ist, was insbesondere bei stark wirksamen Wirkstoffen, wie Paclitaxel unerwünscht ist.

Die in der Beschreibung der Ausführungsbeispiele dargestellten erfindungsgemäßen Ausgestaltungen sind ebenfalls auf die vorstehende Beschreibung der Erfindung, insbesondere ihrer bevorzugten Ausgestaltungen anzuwenden.

## Patentansprüche

1. Implantat zur Implantation in einen menschlichen oder tierischen Organismus umfassend oder bestehend aus einem Aufbau:
a) einem Implantatgrundkörper,
b) einer Primerschicht, die ganz oder teilweise auf der Oberfläche des Implantates aufgebracht ist,
c) einer Wirkstoffschicht bestehend aus ein, zwei, drei oder mehreren Wirkstoffen, die ganz oder teilweise auf der Oberfläche der Primerschicht b) aufgebracht ist und
d) einer diffusionskontrollierenden Schicht, die ganz oder teilweise auf die Wirkstoffschicht c) und gegebenenfalls die Primerschicht b) aufgebracht ist und die Diffusion des oder der Wirkstoffe der Wirkstoffschicht c) kontrolliert.

2. Implantat gemäß Anspruch 1, **dadurch gekennzeichnet, dass** die Primerschicht ausgewählt wird aus ein oder mehreren Materialien aus der Gruppe bestehend aus Siliziumkarbid (SiC), Diamond-Like Carbon (DLC), pyrolytischem Kohlenstoff (pyC), Parylene, Glycocalix und Hydroxylapatit oder Kombinationen hiervon.

3. Implantat gemäß Anspruch 1 oder 2, **dadurch gekennzeichnet, dass** die diffusionskontrollierende Schicht d) ein oder mehrere Polymere umfasst oder daraus besteht, die ausgewählt werden aus der Gruppe bestehend aus Polyestern, vorzugsweise Polydioxanon, Polyglycolid, Polycaprolacton, Polyhydroxyvaleriansäure, Polyhydroxybuttersäuren, Polylactide und Copolymere; Polyurethanen; Silikonen; Polyorganophosphazenen; Polymethacrylaten; Polystyren Block-Copolymeren, Polyethylen-co-vinylacetat; Polyethylenterephthalat sowie Blends oder Copolymere hiervon, sowie Phosphorylcholin-Polymeren und Polysacchariden, vorzugsweise Chitosan, Hyaluronsäure, Heparin, Dextran und Cellulose.

4. Implantat gemäß einem der Ansprüche 1 bis 3, **dadurch gekennzeichnet, dass** der oder die Wirkstoffe der Wirkstoffschicht c) unabhängig voneinander ausgewählt werden aus der Gruppe bestehend aus Antiphlogistika, vorzugsweise Dexamethason, Methylprednisolon und Diclophenac; Cytostatika, vorzugsweise Paclitaxel, Colchicin, Actinomycin D und Methotrexat; Immunsuppressiva, vorzugsweise Limus-Verbindungen, weiter bevorzugt Sirolimus (Rapamycin), Zotarolimus (Abt-578), Tacrolimus (FK-506), Everolimus, Biolimus, insbesondere Biolimus A9 und Pimecrolimus, Cyclosporin A und Mycophenolsäure; Thrombozytenaggregationshemmer, vorzugsweise Abciximab und Iloprost; Statine, vorzugsweise Simvastatin, Mevastatin, Atorvastatin, Lovastatin, Pitavastatin und Fluvastatin; und Estrogene, vorzugsweise 17β-Estradiol, Daizein und Genistein; Lipidregulatoren, vorzugsweise Fibrate; Immunsuppressiva; Vasodilatatoren, vorzugsweise Satane; Calciumkanalblocker; Calcineurininhibitoren, vorzugsweise Tacrolimus; Antiinflammatorika, vorzugsweise Imidazole; Antiallergika; Oligonucleotide, vorzugsweise Decoy-Oligodesoxynukleotid (dODN); Endothelbildner, vorzugsweise Fibrin; Steroide; Proteine/Peptide; Proliferationshemmer; Analgetika und Antirheumatika.

5. Implantat gemäß einem der Ansprüche 1 bis 4, **dadurch gekennzeichnet, dass** der Implantatgrundkörper a) ausgewählt wird aus einer Gruppe bestehend aus Herzschrittmacher; Hirnschrittmacher; Herzimplantate; Schrittmacherelektroden; Defibrillationselektroden; Cochleaimplantate; Implantate für die Zahnmedizin; Depotimplantate, die dazu dienen, ein Depot eines Wirkstoffs zu bilden; biodegradierbare oder dauerhafte, koronare oder periphere Stents; biodegradierbare oder dauerhafte Stents für andere Hohlräume, vorzugsweise die Speiseröhre, den Gallengang, die Harnröhre, die Prostata oder die Luftröhre; und local drug delivery Implantate, die vorzugsweise endovaskulär im Blut oder anderen Hohlräumen implantiert werden.

6. Implantat gemäß einem der Ansprüche 1 bis 5, **dadurch gekennzeichnet, dass** der Implantatgrundkörper ausgewählt wird aus einer Gruppe bestehend aus dauerhaften oder biodegradierbaren koronaren Stents.

7. Verfahren zur Herstellung eines Implantates gemäß einem der vorstehenden Ansprüche, **dadurch gekennzeichnet, dass**
a) folgende Materialien bereit gestellt werden
i. ein Implantatgrundkörper,
ii. eine Zubereitung für die Primerschicht umfassend ein oder mehrere Primermaterialien,
iii. eine Zubereitung für die Wirkstoffschicht umfassend oder bestehend aus ein, zwei, drei oder mehreren Wirkstoffen und
iv. eine Zubereitung für die diffusionskontrollierende Schicht umfassend oder bestehend aus ein oder mehreren Polymeren sowie einem oder mehreren Lösungsmitteln,
b) der Implantatgrundkörper i) ganz oder teilweise mit der Zubereitung für die Primerschicht ii) beschichtet wird,
c) anschließend der ganz oder teilweise mit Primer beschichtete Implantatgrundkörper aus b) ganz oder teilweise mit der Zubereitung für die Wirkstoffschicht iii) beschichtet wird, und
d) anschließend der ganz oder teilweise mit wirkstoffhaltiger Schicht beschichtete Implantatgrundkörper aus c) ganz oder teilweise mit der Zubereitung für die diffusionskontrollierende Schicht iv) beschichtet wird.
